# EUROPEAN PATENT SPECIFICATION

(11) **EP 1 943 701 B1**
(45) Date of publication and mention of the grant of the patent: **22.12.2010**
(21) Application number: 06795986.6
(22) Date of filing: 11.09.2006
(51) Int. Cl.: G21K 1/02, A61B 6/06

(54) **GRID FOR SELECTIVE ABSORPTION OF ELECTROMAGNETIC RADIATION AND METHOD FOR ITS MANUFACTURE**
GITTER FÜR SELEKTIVE ABSORPTION ELEKTROMAGNETISCHER STRAHLUNG UND HERSTELLUNGSVERFAHREN DAFÜR
GRILLE DESTINEE A L'ABSORPTION SELECTIVE D'UN RAYONNEMENT ELECTROMAGNETIQUE ET SON PROCEDE DE FABRICATION

(30) Priority: 19.09.2005 EP 05108574
(43) Date of publication of application: 16.07.2008
(73) Proprietor: Philips Intellectual Property & Standards GmbH, 20099 Hamburg (DE); Koninklijke Philips Electronics N.V., 5621 BA Eindhoven (NL)
(72) Inventor: DORSCHEID, Ralf, 52066 Aachen (DE); VOGTMEIER, Gereon, 52066 Aachen (DE); STEADMAN, Roger, 52066 Aachen (DE); GROOM, Roy K., 52066 Aachen (DE); ELGALY, Avner, 52066 Aachen (DE)
(74) Representative: Schouten, Marcus Maria
(86) International application number: PCT/IB2006/053208
(87) International publication number: WO 2007/034352

(56) References cited:
- WO-A-2005/027143
- DE-A1- 4 217 934
- US-A1- 2003 021 379

## Description

Grids for selective absorption of electromagnetic radiation are known particularly for use in computed tomography (CT) scanners or for use in nuclear imaging devices (gamma cameras, SPECT, PET). Such grids have radiation absorbing lamellae that are oriented so that a transmission direction for electromagnetic radiation (e.g. X-rays, gamma rays, electrons, alpha particles etc.) is defined. Radiation impinging on the grid with a propagation direction different to the transmission direction is predominantly absorbed. The absorption efficiency thereby depends on geometrical parameters such as height of grid, distance between the lamellae, thickness of the lamellae etc. and on other parameters such as the energy of the radiation and the material of the lamellae. Radiation propagating in the transmission direction is essentially fully transmitted (a certain percentage is absorbed due to the geometrical fill factor of the absorbing lamellae).

In a CT scanner the radiation is generated in an X-ray tube. The grid is typically arranged between an object to be irradiated and a detector for detecting the radiation after interaction with the object. The absorbing lamellae are oriented (focused) so that radiation emitted from the focal spot of the X-ray tube (primary radiation) is transmitted and radiation that has undergone a direction changing scattering event in an irradiated object (scattered radiation) is absorbed.

It is an object to manufacture such grids as precisely and efficiently as possible. For a high efficiency a high-aspect ratio - a high ratio of the height of the lamellae in the transmission direction with respect to the thickness of the lamellae - is required. Lamellae with high-aspect ratios are prone to vibrations due to forces acting on them, e.g. when used in the rotating part of a CT scanner. As in CT the grids are usually precisely aligned with the detector elements of the detector, vibrations lead to changes in the grid performance that cannot be calibrated. In case of vibrations of the lamellae, the fraction of X-rays measured by a given detector element varies due to e.g. shadowing effects and the CT image will show artifacts resulting from the vibrations. For radiation grids it is therefore an aim to reduce the proneness of the lamellae to vibration.

Patent document US 6,744, 852 B2 proposes to arrange a carrier material - particularly a polymethacrylimide high-resistance foam -, which is essentially transparent to the radiation, between the lamellae. US 6,744, 852 B2 proposes to form slits in a larger block of carrier material in which the lamellae are to be inserted. In such a grid, the step of inserting the lamellae into the slits becomes tedious for high-aspect lamellae, as the lamellae tend to get stuck in the slits and bend or fold. Another disadvantage of such grids is that there is the need to physically intersect the lamellae to form a precisely manufactured two-dimensional grid. Physical intersection typically requires sawing slits into the lamellae, which is a rather problematic manufacturing step as the lamellae are usually made of a strong metal.

DE4217934A1 discloses a reinforced building element made from a foam material for use in building and packaging. A plurality of steel slats or tapes, which have a height that is larger than a thickness, are inserted into the foam material to increase the strength of the building element.

US2003/021379A1 discloses an anti-scatter grid for an X-ray device which serves to reduced scattered radiation generated in an object to be examined. The anti-scattergrid includes a plurality of absorber laminations for the absorbance of the scattered radiation and a channel medium, in particular a non-elastic high-resistance foam, which is transparent to X-rays and arranged between the absorber laminations. The absorber laminations are provided into a set of slits in a first side of the channel medium.

WO2005/027143A discloses an arrangement for collimating electromagnetic radiation comprising a macro-collimator, which has at least to cut-outs, and micro-collimator structures, which are positioned in the cut-outs of the macro-collimator and have lamellae that absorb electromagnetic radiation, so that collimator channels are formed which in each case extend such that they are transparent in a transmission direction.

It is therefore an object of the present invention to provide a grid and a method of manufacturing such a grid that is improved with respect to the known grids and the known manufacturing methods.

The object of the invention is achieved by a radiation absorption arrangement adapted for selective absorption of electromagnetic radiation according to clami 1. The arrangement comprises a block of a rigid foam material, the foam material being essentially transparent to the electromagnetic radiation, a first set of radiation absorbing lamellae, and a second set of radiation absorbing lamellae, the first and the second set of lamellae being arranged in the block of foam material so that a radiation transmission direction is defined .The first set of lamellae is arranged in a first set of cavities that extend into the foam material into the radiation transmission direction from a radiation entrance side of the block of foam material, which side faces in an operating state a radiation source, and the second set of lamellae is arranged in a second set of cavities that extend into the foam material into the radiation transmission direction from an exit side of the block of foam material. The first and the second set of lamellae are arranged such that there is a gap between the first set of lamellae and the second set of lamellae that extends into the transmission direction and that is located around a center line in the block of foam material. In such a radiation absorption arrangement, it is an advantage, that the block carrier material defines the outer dimensions of the radiation absorption arrangement and that the first and second set of lamellae can be arranged in the radiation absorption arrangement with reference to the outer dimensions, so that a radiation absorption arrangement of high precision is formed that is stable and where the lamellae are fixed by the carrier material. The carrier material itself is essentially transparent to electromagnetic radiation, so that the performance of the radiation absorption arrangement is essentially only determined by the absorption efficiency of the lamellae and not deteriorated by absorption in the carrier material. Typical lamellae are made of a high-Z metal, where Z is the atomic number, e.g. molybdenum, tungsten or lead. Depending on the energy of the electromagnetic radiation to be absorbed, the lamellae could also be made of lower Z material, e.g. copper or iron. Instead of being made of sheet metal or the like, the lamellae could also be manufactured by a plastic injection molding process, where metal powder is mixed with the plastic. The cavities can be formed e.g. by sawing using a disc saw or a wire saw or by any other suitable machining process. It is then simple to machine the cavities and to arrange the lamellae in the cavities even for focused radiation absorption arrangements, as they are fully independent of each other. The cavities could also have different dimensions for accommodating different sized lamellae, which means that the cavities could e.g. have different depth values (measured in transmission direction) and/or thickness values.

In one embodiment of the invention, the first set of lamellae is arranged in a crossed fashion with respect to the second set of lamellae. Thus, a two-dimensional structure is formed without the need to physically intersect the lamellae. As the foam material serves as a carrier for the lamellae, no problems arise with respect to fixation of the lamellae on the sides. Also, a plurality of several relatively small radiation absorption arrangements can thus be positioned in a (curved) matrix arrangement so that a large radiation absorption arrangement covering a large size detector can be formed as each small radiation absorption arrangement is stable in itself.

In one embodiment of the invention, the lamellae are glued into the cavities. In contrast to clamping, this fixes the lamellae strongly so that they do not loosen their fixation even in operation in a rotating gantry.

In another embodiment of the invention, the lamellae are arranged parallel to each other. This allows selective absorption of all radiation that impinges at an oblique angle on the radiation absorption arrangement.

In an even further embodiment of the invention, the lamellae of the first set of lamellae are aligned with the lamellae of the second set of lamellae. Instead of inserting long lamellae into the foam material to manufacture a high aspect ratio radiation absorption arrangement, only about two times shorter lamellae need to be inserted, which reduces problems associated with bending and folding of the lamellae during the insertion process. It is also envisaged to have lamellae of different sizes, e.g. the first set of lamellae could be longer in transmission direction than the second set of lamellae and/or the second set of lamellae could be thicker than the first set of lamellae.

The invention further relates to a detector arrangement for detecting electromagnetic radiation that comprises a detector and a radiation absorption arrangement according to the invention. The invention furthermore relates also to a medical imaging device that comprises a detector arrangement according to the invention.

The object of the invention is also solved by a method of manufacturing a radiation absorption arrangement adapted for selective absorption of electromagnetic radiation asd defined by claim 8. An advantageous embodiment is defined by claim 9.

The invention is further elucidated in the following by describing various embodiments and referring to drawings.

In the drawings
Fig. 1 shows a side view of a first step of an exemplary method of manufacturing a grid according to the invention, namely the step of providing a block of rigid foam material for further treatment,
Fig. 2 shows a side view of a second step of an exemplary method of manufacturing a grid according to the invention, namely the forming of a first set of cavities in the block of foam material,
Fig. 3 shows a side view of a third step of an exemplary method of manufacturing a grid according to the invention, namely the forming of a second set of cavities in the block of foam material,
Fig. 4 shows a side view of a fourth step of an exemplary method of manufacturing a grid according to the invention, namely the arrangement of a first set of radiation absorbing lamellae into the first set of cavities,
Fig. 5 shows a top view of the scenario as shown in the side view depicted in Fig. 4,
Fig. 6 shows a side view of a grid according to an embodiment of the invention, where the lamellae of the first set of radiation absorbing lamellae and the lamellae of the second set of radiation absorbing lamellae are arranged in a parallel fashion and are aligned with each other,
Fig. 7 shows a side view of a grid according to another embodiment of the invention, where the lamellae of first set of radiation absorbing lamellae and the lamellae of the second set of radiation absorbing lamellae are arranged in a focused fashion and are aligned with each other,
Fig. 8 shows a perspective view of a block of foam material in which a first and a second set of cavities are formed in a focused fashion and are arranged crossed to each other,
Fig. 9 shows a top view of a grid according to the invention made in a two-dimensional fashion where the lamellae of the first and the second set of lamellae are depicted for the sake of clarity,
Fig. 10 shows a schematic depiction of a nuclear medical imaging device (e.g. a SPECT scanner) with a detector having four grids, and
Fig. 11 shows a schematic depiction of a CT device having an X-ray source and a detector having a focused grid.

Radiation absorption grids are widely used, e.g. in medical imaging devices, like computed tomography (CT) scanners or in single photon emission computed tomography (SPECT), or in analytical equipment, e.g. in X-ray diffraction analysis devices. In these devices radiation absorption grids are used to absorb radiation quanta that impinge on the grid with a propagation direction different from a (where required locally variable) transmission direction. In order to achieve this, the grids comprise radiation-absorbing lamellae that are oriented in alignment with a (locally variable as required ) transmission direction. Thus, transmission channels are formed between the lamellae.

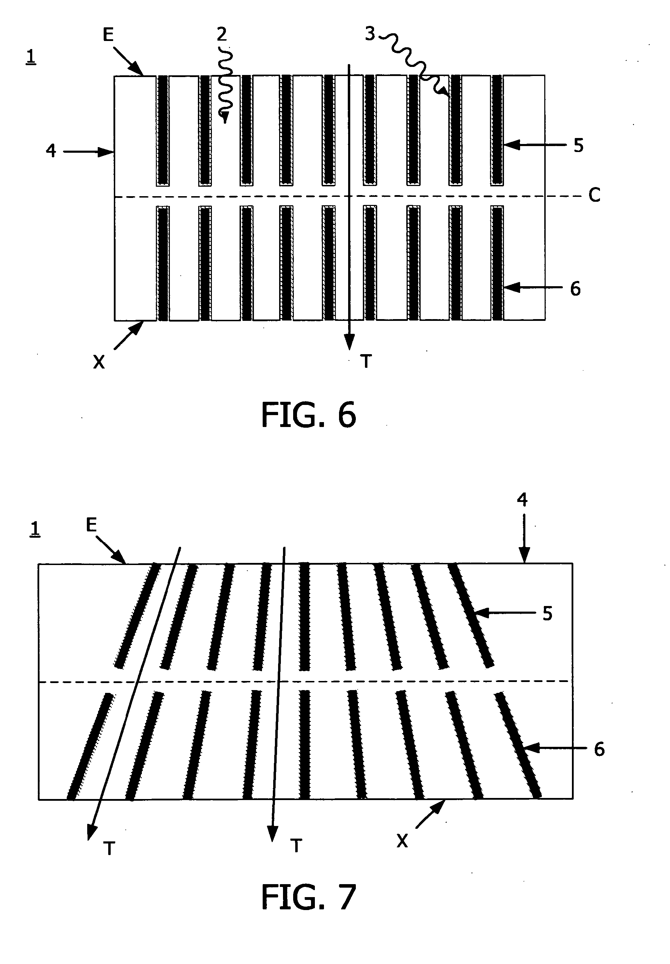

In one typical embodiment, the lamellae are oriented parallel to each other, which results in a transmission direction that is constant over the whole grid extension (see Fig. 6 and the explanations referring to Fig. 6). In another typical embodiment, the lamellae are focused, so that the locally variable transmission directions intersect at a focal spot point (see Fig. 7 and the explanations referring to Fig. 7). The latter arrangement is e.g. used in CT, where the lamellae are focused on the focal spot of the X-ray source.

In CT the grid is positioned in front of a detector that measures the radiation that has traversed an irradiated object. Hence, X-ray quanta that are scattered in the irradiated object are predominantly absorbed by the focused lamellae, in case the scattering process has changed the propagation direction of the quanta not insufficiently. The larger the irradiated object volume becomes the higher gets the ratio between scattered radiation and primary radiation that has undergone no scattering event. The irradiated object volume increases with object size and also with the cone width of the X-ray beam.

The typical sizes of a lamella depend on the specific application. For medical applications the lamella dimensions are typically in the following ranges: thickness (measured perpendicularly to the transmission direction) d = 10 - 500 µm and height (measured in transmission direction) h = 1 - 50 mm. The length of the lamellae depends on the application and can be as long as 1 = 400 mm for a radiography grid.

The material of the lamellae depends on the energy of the radiation to be absorbed. For low energy radiation, e.g. as used in mammography, where radiation quanta have a mean energy of about 20 keV, the radiation absorption coefficients of e.g. copper or iron suffice even for a lamella thickness of 10 - 50 µm. For higher mean radiation energies (e.g. for radiography, where the mean energy is typically about 75 keV, or for CT, where the mean energy is typically about 100 - 140 keV) the lamella material is chosen from stronger absorbing metals e.g. molybdenum, tungsten, or lead.

Grids that are used for radiography are made from lead lamellae that e.g. have exemplary thickness of d = 30 µm, a height of h = 2 mm, and a length of 1 = 30 cm. In order to stabilize such a grid, the transmission channels are filled with a channel material, e.g. aluminum or paper. The channel material is chosen to absorb as little radiation as possible to achieve high grid efficiency. In CT exemplary dimensions for a lamella could be d = 100 µm, h = 20 mm, and 1 = 30 mm. These lamellae are fixed at their sides and the channel material is air. For SPECT a two-dimensional structure instead of a one-dimensional arrangement of lamellae is used. Exemplary dimensions for SPECT are e.g. d = 500 µm and h = 40 mm over a total grid size of 40 x 40 cm². A SPECT grid is usually manufactured using a lead casting process. Typically, the two-dimensional structure consists of hexagonal channels, so that each lamella has a length of only a few millimeters.

For applications like CT or radiography, the need for two-dimensional grids is immense. For CT this is triggered by the growing X-ray beam cone sizes. In the past few years, the cones were widened from a maximum value of about 2 cm at detector level to about 10 cm at detector level. A cone of 40 cm at detector level would suffice to perform a full cardiac or liver scan in a single circular scan. As has been explained above, such an increase of the cone sizes leads to an increase in the ratio between scattered radiation and transmitted (or primary) radiation (S/P ratio). Standard linear grids, where the lamellae are arranged one-dimensionally, cannot cope with such an increase in the S/P ratio. The high S/P ratio leads to artifacts in the CT images and also to an increase in the image noise.

In the following, various exemplary manufacturing methods and embodiments of grids according to the invention are described. The grids according to the invention combine ease of manufacture, precision, high efficiency and stability. Grids according to the present invention can be parallel or focused, they can be one-dimensional or two-dimensional.

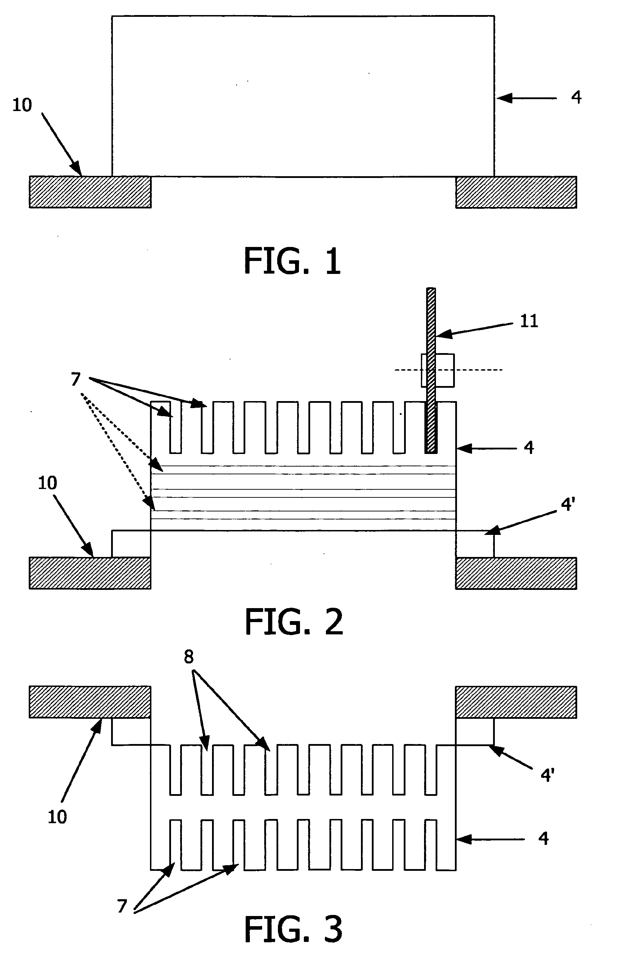

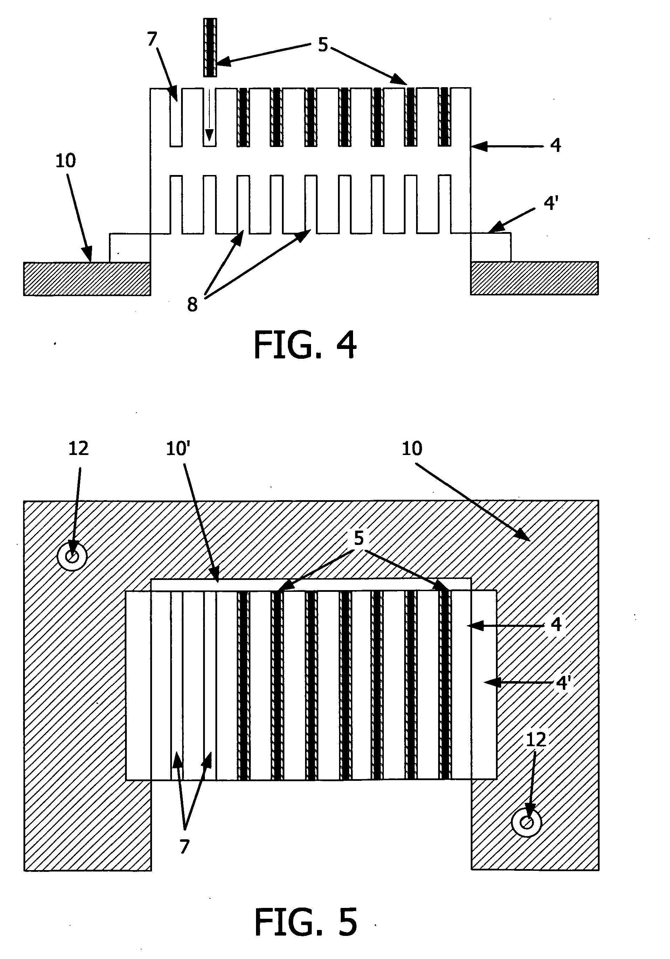

Fig. 1 shows in a side view a first step in an exemplary manufacturing method of making a grid according to the invention. A block of a rigid foam material 4 is fixed to a base plate 10, e.g. by means of a hot wax. This allows to fixedly connect the block of foam material 4 to the base plate 10 and to allow for further mechanical treatment of the block of foam material 4 without moving the block of foam material 4 relative to the base plate 10. Hence, the base plate 10 defines a reference coordinate system for precise structuring of the block of foam material 4. In the exemplary manufacturing method shown the base plate 10 has an opening 10' (see Fig. 5 for a depiction of the opening 10' in a top view). In the embodiment shown, the opening 10' is U-shaped (which renders the base plate 10 to be C-shaped) and allows access to the side of the block of foam material 4 that is connected to the base plate. A typical foam material is ROHACELL®, a polymethacrylimid (PMI) hard foam, but other hard and lightweight foam materials of similar mechanical rigidity can likewise be used. ROHACELL® has also adequate thermal properties and does not deteriorate under high X-ray doses.

In Fig. 2 a second step in the exemplary manufacturing method for a grid according to the invention is shown. At the shown stage of the manufacturing process the outer shape of the block of foam material was already defined, e.g. by milling or sawing or any other adequate method. As the milling tools or the saw can be precisely positioned with respect to the base plate 10 (see Fig. 5 for a depiction of pass holes 12 for a precise fixation of the base plate 10 to a backbone structure to which also the tools can be fixed or at least precisely referenced to by means of calibration), the outer shape is precisely defined in the reference system of the base plate 10. Here, those parts 4' of the block of foam material that are fixed to the base plate 10 with hot wax are dispensed with after the grid has been manufactured - e.g. by simply breaking them off. This ensures that the final grid is not contaminated with wax, which would absorb radiation more strongly than the foam material. In Fig. 2 is also shown that a first set of cavities 7 (slits in the present embodiment) has been formed in the block of foam material 4. A disc saw 11 for sawing the first set of cavities 7 is schematically shown. As the outer shape of the block of foam material 4 has been structured within the coordinates of the reference system, the cavities are also formed with precise reference to the outer shape of the block of foam material 4. Hence, the outer shape of the block of foam material can be used for precise alignment of the final grid with other structures, particularly to align the grid with a pixilated detector in such a way that the lamellae are aligned with the inactive border areas of the detector pixels. This is a great advantage of the disclosed method.

In contrast to vertically sawing the cavities 7 into the foam material, it is also possible to horizontally saw the cavities 7, as is indicated in dashed lines in Fig. 2.

In Fig. 3 a third step in the exemplary manufacturing method for a grid according to the invention is shown. At this stage, the base plate 10 had been disconnected, vertically rotated and again fixed to the backbone structure (using the pass holes 12 as depicted in Fig. 5 for a precise fixation) so that the saw could access the other side of the block of foam material for sawing a second set of cavities 8 into the foam material. In case the cavities 7 were sawed horizontally as explained above, the base plate 10 would be horizontally rotated and the second set of cavities 8 would also be sawed horizontally. The horizontal sawing has the advantage that a disc saw 11 with a lower diameter could be used, as the saw would not need to cover the thickness of the base plate 10 and any additional space due to the outer shaping of the block of foam material 4. The smaller the diameter of the disc saw 11 the more precise the cavities 7, 8 can be sawed. Instead of a disc saw 11 other means known to a skilled person could be used, e.g. a wire saw.

In Fig. 4 a fourth step in the exemplary manufacturing method for a grid according to the invention is shown. At this stage lamellae from a first set of radiation absorbing lamellae 5 are arranged in the first set of cavities 7. Advantageously, the lamellae are fixed in the cavities by means of glue, e.g. an epoxy resin. An epoxy resin for optical applications such as EPO-TEC 301 from Polytec PT GmbH Polymere Technologien, Waldbronn, Germany, fulfills the requirements to be able to glue molybdenum and ROHACELL® while being stable under high doses of ionizing radiation. This could be achieved by e.g. first sliding a lamella into a cavity and then pouring some low-viscosity glue into the cavity. As - due to tolerance reasons - the thickness of the cavities needs to be somewhat larger than the thickness of the lamellae, the low viscosity glue flows into the remaining space and fixes the lamella in the cavity after it has hardened. In another embodiment of this step, the lamella is provided with a printed coating of glue and then slit into the cavity. Other possibilities obvious to the skilled person are also possible.

Fig. 5 shows a top view on the grid in the stage as shown in Fig. 4. The base plate 10 has an opening 10' over which the block of foam 4 material was arranged. The parts 4' of the block of foam material that remain after the outer shape of the block of foam material had been formed are fixed to the base plate, e.g. by a hot wax, as explained above. Seven lamellae from the first set of radiation absorbing lamellae 5 are already glued into the slits, while two empty cavities from the first set of cavities 7 remain. The base plate 10 has pass holes 12 for a precise connection of the base plate 10 to a backbone structure (not shown). Tools like a milling tool, a saw etc. can also be fixed to the backbone structure or can be referenced to the backbone structure by means of a calibration. This allows to precisely shape the block of foam material in the reference coordinate system defined by the backbone structure. Due to the precise connection of the base plate 10 to the backbone structure via the pass holes 12, the base plate 10 can be vertically or horizontally rotated or can be translated to another position on the backbone structure while preserving the precise reference to the block of foam material 4. This means that a cavity (in the embodiment whown the cavity is manufactured as a slit) can be sawed as precisely (with reference to the outer geometry of the block of foam material 4) in the block of foam material with the base plate 10 being in a first position as another cavity in a second position (that is rotated or translated to the first position). This only requires the precise manufacture of the base plate 10, the pass holes 12 and any positioning pins or the like in the backbone structure. An overall precision of the block of foam material and the slits of about ± 10 µm can be achieved using aluminum as a base plate and backbone structure material and utilizing today's CAD/CAM machines.

Fig. 6 shows a side view of a grid 1 according to one embodiment of the invention. The cross section of the block of foam material 4 is of a rectangular shape. An entrance side E of the grid 1 faces in an operating state a radiation source. A first set of radiation absorbing lamellae 5 is arranged in the block of foam material 4 in cavities that extend from the entrance side E into the block of foam material close to the center line C. The lamellae of the first set of lamellae 5 are arranged parallel to each other and perpendicular to the entrance E. In Fig. 6 is shown that the lamellae of the first set of lamellae 5 are flush with the entrance side E. In other embodiments of the invention the lamellae could of course also project over the entrance side E. A second set of lamellae 6 is arranged in a second set of cavities that extend from an exit side X of the block of foam material into the block of foam material close to the center line C. In the embodiment shown, the second set of lamellae 6 is aligned with the first set of lamellae 5, so that the first set of lamellae operates essentially as an extension of the first set of lamellae. The gap between them around the center line C is negligible when considering the overall efficiency of the grid. It is clear that in such a grid the lamellae need not be inserted into the cavities over the full aimed height of an effective lamella but only about half the effective lamella height. This reduces the problems associated with bending and folding of the lamellae. Due to the alignment of the lamellae a transmission direction T is defined. This transmission direction T is constant over the extension of the grid as the lamellae are arranged parallel to each other. An electromagnetic radiation quantum 2 that impinges on the grid having a propagation direction identical to the transmission direction T will traverse the grid without being absorbed. Only those quanta that impinge on the topside of a lamella will be absorbed; this fraction is given by the geometrical fill factor of the lamellae. E.g. if the lamellae have a thickness of d = 100 µm and are arranged at a center-to-center distance of 1 mm the geometrical fill factor of the lamellae is 10%. A quantum 3 that has a propagation direction not only insignificantly different from the transmission direction T will be absorbed with high probability. The absorption probability depends on the geometrical dimensions, the lamella material, the energy of the quantum and the propagation direction of the quantum. Instead of an alignment as shown in Fig. 6, the lamellae of the second set of lamellae 6 can also be positioned in the center between two lamellae of the first set of lamellae 5. This would allow for manufacturing a grid with a high density of lamellae while the thickness of the carrier material between the channels remains as much as about twice the effective distance of the lamellae. The risk of breaking the carrier material during manufacture is thus strongly reduced.

In Fig. 7 another embodiment of a grid according to the invention is shown in a side view. The grid shown in Fig. 7 differs from the grid shown in Fig. 6 in that the lamellae of the first and of the second set of lamellae 5, 6 are focused. This leads to a transmission direction that changes over the extension of the grid 1 as can be seen from the two transmission directions T shown in Fig. 7. In case the lamellae are focused on a focal spot of a radiation source (e.g. an X-ray source) or on a hot spot then the primary radiation will traverse the grid essentially without being absorbed. Radiation quanta that are scattered in an object (not shown) so that their propagation direction is changed will be absorbed in the grid.

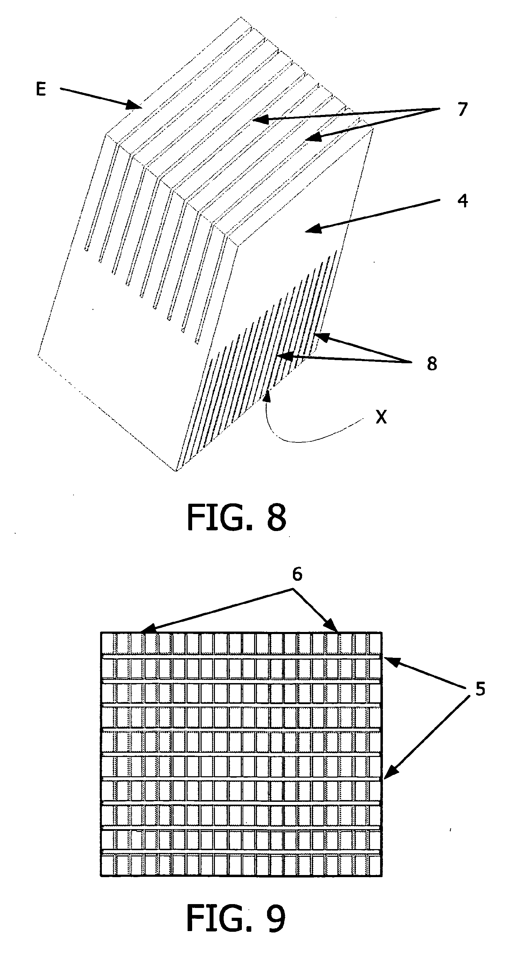

In Fig. 8 a further embodiment of a grid according to the invention is indicated. In Fig. 8 a perspective view of a block of foam material 4 is shown where the first set of cavities 7 is formed in focused fashion and the second set of cavities 8 is likewise formed in a focused fashion but the cavities of the second set of cavities is perpendicularly arranged with respect to the cavities of the first set of cavities. After insertion of the lamellae into the cavities, such an arrangement results in a two-dimensional grid arrangement, where the lamellae of the first and the second set of lamellae form individual channels when viewed in transmission direction. Even though at least twice the height is required for such a two-dimensional grid when compared to e.g. a cast lead grid as used in nuclear medicine devices, the present embodiment of a grid according to the invention overcomes the problem of meshing the lamellae as they do not need to be physically intersected. The foam material stabilizes the lamellae. The grid can be seen as one module of a large grid, so that a large area can be covered with a number of grid modules positioned in a matrix-like arrangement.

Fig. 9 shows a top view of the lamellae of the grid of Fig. 8 after they have been inserted into the cavities. For the sake of clarity the first set of lamellae is shown as well as the second set of lamellae to better show the two-dimensional grid structure that has been achieved. This two-dimensional grid according to the invention has been achieved without the need to saw through the first set of lamellae to insert the second set of lamellae. There is e.g. no risk that the lamellae fray out when they are sawed through.

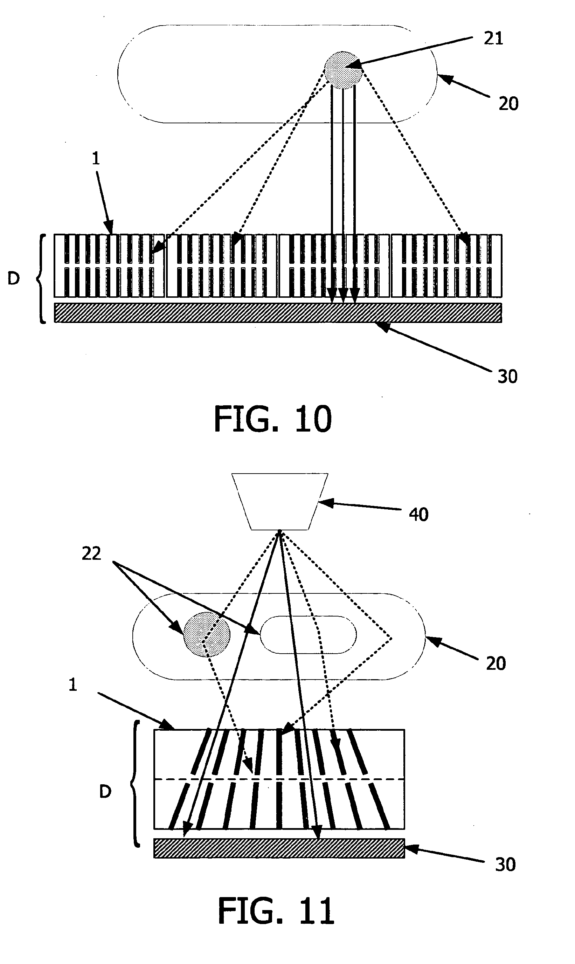

In Fig. 10 a schematic depiction of a nuclear medical (NM) imaging device (e.g. a gamma camera or a SPECT scanner) is given. The main feature of an NM device is a detector arrangement D for detecting radiation emitted from an object 20 (e.g. a patient or an animal). The detector arrangement consists of a detector 30 for detecting electromagnetic radiation and at least one grid 1 for selective absorption of electromagnetic radiation. Further features of the NM device, e.g. a gantry to which the detector arrangement is mounted, are not shown. In NM imaging, the object receives an injection of a radioactive isotope, typically a gamma emitter or a positron emitter (which will lead to two emitted quanta after annihilation of the positron). The radioactive isotope is typically attached to a molecule that specifically attaches to a certain type of tissue, e.g. heart tissue or cancerous tissue. In Fig. 10 a radiation emitting region 21 is indicated. It is then of interest to achieve an image of the body to identify the regions to which the radioactively labeled molecules have attached. For this purpose a parallel grid 1 as shown in Fig. 10 is required. A parallel grid 1 allows for achieving planar images of the object 20 where the radiation emitting regions 21 are imaged as sharp areas. Radiation emitted from the radiation emitting regions in transmission direction of the grids is essentially transmitted (indicated by full lines) and radiation impinging on the grid at an inclined angle will be absorbed (indicated by dashed lines). In the exemplary embodiment shown, the detector arrangement D is equipped with four grids 1, but a detector arrangement could also have one grid or e.g. 16 grids arranged in a 4 x 4 matrix over the detector 30.

In Fig. 11 a schematic depiction of a CT device is given. The CT device comprises an exemplary detector arrangement D having a detector 30 and a focused grid 1. In another embodiment, the detector arrangement has a curved detector and several focused grids mounted on top of the detector. The grids are focused on the focal spot of a radiation source 40. This leads to the absorption of radiation scattered in the object 20 (indicated by dashed lines) and to the transmission of primary radiation (indicated by full lines). Hence, a CT detector images the attenuation properties along the line between the focal spot and a respective detector element. Dense objects regions 22 having higher attenuation properties will lead to a stronger absorption than other, low absorbing regions of the object.

## Claims

1. Radiation absorption arrangement (1) adapted for selective absorption of electromagnetic radiation (2, 3), the arrangement comprising:
- a block of a rigid foam material (4), the foam material being essentially transparent to the electromagnetic radiation (2, 3),
- a first set of radiation absorbing lamellae (5), and
- a second set of radiation absorbing lamellae (6),
the first and the second set of lamellae (5, 6) being arranged in the block of foam material (4) so that a radiation transmission direction (T) is defined,
wherein the first set of lamellae (5) is arranged in a first set of cavities (7) that extend into the foam material into the radiation transmission direction (T) from a radiation entrance side (E) of the block of foam material (4), which side faces in an operating state a radiation source, and wherein the second set of lamellae (6) is arranged in a second set of cavities (8) that extend into the foam material into the radiation transmission direction (T) from an exit side (X) of the block of foam material (4) and **characterised in that** the first and the second set of lamellae (5, 6) are arranged such that there is a gap between the first set of lamellae (5) and the second set of lamellae (6) that extends into the transmission direction (T) and that is located around a center line (C) in the block of foam material (4).

2. A radiation absorption arrangement according to claim 1, wherein the first set of lamellae (5) is arranged in a crossed fashion with respect to the second set of lamellae (6).

3. A radiation absorption arrangement according to any one of claims 1 to 2, wherein the first set of lamellae (5) and the second set of lamellae (6) are fixed to the foam material by means of a glue material (9), particularly an electromagnetic radiation resistant glue.

4. A radiation absorption arrangement according to any one of claims 1 to 3, wherein the lamellae of the first set of lamellae (5) are arranged parallel to each other.

5. A radiation absorption arrangement according to any one of claims 1 to 4, wherein the lamellae of the second set of lamellae (6) are arranged in alignment with the lamellae of the first set of lamellae (5) so that the second set of lamellae (6) operates essentially as an extension of the first set of lamellae (5).

6. A detector arrangement (D) for detecting electromagnetic radiation comprising a detector (30) and a radiation absorption arrangement (1) adapted for selective absorption of electromagnetic radiation (2, 3) according to any one of claims 1 to 5.

7. A medical imaging device comprising a detector arrangement (D) according to claim 6.

8. A method of manufacturing a radiation absorption arrangement adapted for selective absorption of electromagnetic radiation, the method comprising the steps of
a) providing a block of a rigid foam material (4), the foam material being essentially transparent to the electromagnetic radiation,
b) forming a first set of cavities (7) into the block of foam material (4) extending from an entrance side (E) of the block of foam material (4), which side faces in an operating state a radiation source, so that the first set of cavities (7) extends into a radiation transmission direction (T),
c) forming a second set of cavities (8) into the block of foam material (4) extending from an exit side (X) of the block of foam material (4) into the radiation transmission direction (T) such that there is a gap between the first set of lamellae (5) and the second set of lamellae (6) that extends into the radiation transmission direction (T) and that is located around a center line (C) in the block of foam material (4) between the first set of cavities (7) and the second set of cavities (8) , and
d) arranging a first set of radiation absorbing lamellae (5) in the first set of cavities (7) and a second set of radiation absorbing lamellae (6) in the second set of cavities (8).

9. A method according to claim 8, wherein the block of rigid foam material (4) is fixed to a base plate (10) and the base plate (10) is fixed to a backbone structure via pass holes (12) in the base plate (10), and wherein before step c) and after step b) the base plate (10) is disconnected from the backbone structure, whereafter the base plate is rotated horizontally or vertically, and whereafter the base plate (10) is again fixed to the backbone structure using the pass holes (12).

## Patentansprüche

1. Strahlungsabsorptionsanordnung (1) zur selektiven Absorption von elektromagnetischer Strahlung (2, 3), wobei die Anordnung Folgendes umfasst:
- einen Block aus starrem Schaummaterial (4), wobei das Schaummaterial im Wesentlichen transparent für die elektromagnetische Strahlung (2, 3) ist,
- einen ersten Satz Strahlung absorbierender Lamellen (5), und
- einen zweiten Satz Strahlung absorbierender Lamellen (6),
wobei der erste und der zweite Satz von Lamellen (5, 6) so in dem Schaummaterialblock (4) angeordnet sind, dass eine Strahlungstransmissionsrichtung (T) definiert wird,
wobei der erste Satz von Lamellen (5) in einem ersten Satz von Hohlräumen (7) angeordnet ist, die sich in der Strahlungstransmissionsrichtung (T) von einer Strahlungseintrittsseite (E) des Schaummaterialblocks (4) aus, welche in einem Betriebszustand einer Strahlungsquelle gegenüberliegt, in das Schaummaterial erstrecken, und wobei der zweite Satz von Lamellen (6) in einem zweiten Satz von Hohlräumen (8) angeordnet ist, die sich in der Strahlungstransmissionsrichtung (T) von einer Austrittsseite (X) des Schaummaterialblocks (4) aus in das Schaummaterial erstrecken, und **dadurch gekennzeichnet, dass** der erste und der zweite Satz von Lamellen (5, 6) derartig angeordnet sind, dass sich eine Lücke zwischen dem ersten Satz von Lamellen (5) und dem zweiten Satz von Lamellen (6) befindet, die in der Transmissionsrichtung (T) verläuft und die sich um eine Mittellinie (C) im Schaummaterialblock (4) herum befindet.

2. Strahlungsabsorptionsanordnung nach Anspruch 1, wobei der erste Satz von Lamellen (5) in einer gekreuzten Weise in Bezug auf den zweiten Satz von Lamellen (6) angeordnet ist.

3. Strahlungsabsorptionsanordnung nach einem der Ansprüche 1 bis 2, wobei der erste Satz von Lamellen (5) und der zweite Satz von Lamellen (6) mit Hilfe eines Klebematerials (9), insbesondere eines gegen elektromagnetische Strahlung beständigen Klebers, an dem Schaummaterial befestigt sind.

4. Strahlungsabsorptionsanordnung nach einem der Ansprüche 1 bis 3, wobei die Lamellen des ersten Satzes von Lamellen (5) parallel zueinander angeordnet sind.

5. Strahlungsabsorptionsanordnung nach einem der Ansprüche 1 bis 4, wobei die Lamellen des zweiten Satzes von Lamellen (6) in einer Linie mit den Lamellen des ersten Satzes von Lamellen (5) liegen, so dass der zweite Satz von Lamellen (6) im Wesentlichen als eine Verlängerung des ersten Satzes von Lamellen (5) fungiert.

6. Detektoranordnung (D) zum Detektieren von elektromagnetischer Strahlung mit einem Detektor (30) und einer Strahlungsabsorptionsanordnung (1) zur selektiven Absorption von elektromagnetischer Strahlung (2, 3) nach einem der Ansprüche 1 bis 5.

7. Medizinische Bildgebungsvorrichtung mit einer Detektoranordnung (D) nach Anspruch 6.

8. Verfahren der Herstellung einer Strahlungsabsorptionsanordnung zur selektiven Absorption von elektromagnetischer Strahlung, wobei das Verfahren die folgenden Schritte umfasst:
a) Schaffen eines Blocks aus starrem Schaummaterial (4), wobei das Schaummaterial im Wesentlichen transparent für die elektromagnetische Strahlung ist,
b) Formen eines ersten Satzes von Hohlräumen (7) in dem Schaummaterialblock (4), die von einer Eintrittsseite (E ) des Schaummaterialblocks (4) ausgehen, welche in einem Betriebszustand einer Strahlungsquelle gegenüberliegt, so dass der erste Satz von Hohlräumen (7) in einer Strahlungstransmissionsrichtung (T) verläuft,
c) Formen eines zweiten Satzes von Hohlräumen (8) in dem Schaummaterialblock (4), die sich von einer Austrittsseite (X) des Schaummaterialblocks (4) in die Strahlungstransmissionsrichtung (T) erstrecken, so dass sich eine Lücke zwischen dem ersten Satz von Lamellen (5) und dem zweiten Satz von Lamellen (6) befindet, die in Strahlungstransmissionsrichtung (T) verläuft und die sich um eine Mittellinie (C ) im Schaummaterialblock (4) zwischen dem ersten Satz von Hohlräumen (7) und dem zweiten Satz von Hohlräumen (8) befindet, und
d) Anordnen eines ersten Satzes von Strahlung absorbierenden Lamellen (5) in dem ersten Satz von Hohlräumen (7) und eines zweiten Satzes von Strahlung absorbierenden Lamellen (6) in dem zweiten Satz von Hohlräumen (8).

9. Verfahren nach Anspruch 8, wobei der Block aus starrem Schaummaterial (4) an einer Basisplatte (10) befestigt ist und die Basisplatte (10) über Durchgangslöcher (12) in der Basisplatte (10) an einer Stützstruktur befestigt ist, und wobei die Basisplatte (10) vor Schritt c) und nach Schritt b) von der Stützstruktur getrennt wird, anschließend die Basisplatte horizontal oder vertikal gedreht wird und anschließend die Basisplatte (10) wieder über die Durchgangslöcher (12) an der Stützstruktur befestigt wird.

## Revendications

1. Agencement d'absorption de rayonnement (1) adapté pour une absorption sélective du rayonnement électromagnétique (2, 3), l'agencement comprenant :
- un bloc de matériau mousse rigide (4), le matériau mousse étant essentiellement transparent au rayonnement électromagnétique (2, 3),
- un premier jeu de lamelles absorbant le rayonnement (5), et
- un second jeu de lamelles absorbant le rayonnement (6),
les premier et second jeux de lamelles (5, 6) étant agencés dans le bloc de matériau mousse (4) de sorte que la direction de transmission du rayonnement (T) est définie,
où le premier jeu de lamelles (5) est agencé dans un premier jeu de cavités (7) qui s'étend dans le matériau mousse dans la direction de transmission du rayonnement (T) depuis un côté entrée de rayonnement (E) du bloc de matériau mousse (4), lequel côté est en regard dans un état de fonctionnement d'une source de rayonnement, et dans lequel le second jeu de lamelles (6) est agencé dans un second jeu de cavités (8) qui s'étend dans le matériau mousse dans la direction de transmission du rayonnement (T) depuis un côté sortie (X) du bloc de matériau mousse (4) et **caractérisé en ce que** le premier et le second jeu de lamelles (5, 6) sont agencés de telle sorte qu'il existe un espace entre le premier jeu de lamelles (5) et le second jeu de lamelles (6) qui s'étend dans la direction de transmission (T) et qui se situe autour d'une ligne centrale (C) dans le bloc de matériau mousse (4).

2. Agencement d'absorption de rayonnement selon la revendication 1, dans lequel le premier jeu de lamelles (5) est agencé de façon croisée par rapport au second jeu de lamelles (6).

3. Agencement d'absorption de rayonnement selon l'une quelconque des revendications 1 et 2, dans lequel le premier jeu de lamelles (5) et le second jeu de lamelles (6) sont fixés au matériau mousse au moyen d'un matériau de colle (9), en particulier une colle résistant au rayonnement électromagnétique.

4. Agencement d'absorption de rayonnement selon l'une quelconque des revendications 1 à 3, dans lequel les lamelles du premier jeu de lamelles (5) sont agencées parallèlement les unes aux autres.

5. Agencement d'absorption de rayonnement selon l'une quelconque des revendications 1 à 4, dans lequel les lamelles du second jeu de lamelles (6) sont agencées en alignement avec les lamelles du premier jeu de lamelles (5) de sorte que le second jeu de lamelles (6) fonctionne essentiellement comme une extension du premier jeu de lamelles (5).

6. Agencement de détecteur (D) pour détecter le rayonnement électromagnétique, comprenant un détecteur (30) et un agencement d'absorption de rayonnement (1) adapté pour une absorption sélective du rayonnement électromagnétique (2, 3) selon l'une quelconque des revendications 1 à 5.

7. Dispositif d'imagerie médicale comprenant un agencement de détecteur (D) selon la revendication 6.

8. Procédé de fabrication d'un agencement d'absorption de rayonnement adapté pour une absorption sélective du rayonnement électromagnétique, le procédé comprenant les étapes consistant à
a) fournir un bloc de matériau mousse rigide (4), le matériau mousse étant essentiellement transparent au rayonnement électromagnétique,
b) former un premier jeu de cavités (7) dans le bloc de matériau mousse (4) s'étendant depuis un côté entrée (E) du bloc de matériau mousse (4), lequel côté est en regard dans un état de fonctionnement d'une source de rayonnement, de sorte que le premier jeu de cavités (7) s'étend dans une direction de transmission du rayonnement (T),
c) former un second jeu de cavités (8) dans le bloc de matériau mousse (4) s'étendant depuis un côté sortie (X) du bloc de matériau mousse (4) dans la direction de transmission de rayonnement (T) de telle sorte qu'il existe un espace entre le premier jeu de lamelles (5) et le second jeu de lamelles (6) qui s'étend dans la direction de transmission du rayonnement (T) et qui se situe autour d'une ligne centrale (C) dans le bloc de matériau mousse (4) entre le premier jeu de cavités (7) et le second jeu de cavités (8), et
d) agencer un premier jeu de lamelles absorbant le rayonnement (5) dans le premier jeu de cavités (7) et un second jeu de lamelles absorbant le rayonnement (6) dans le second jeu de cavités (8).

9. Procédé selon la revendication 8, dans lequel le bloc de matériau mousse rigide (4) est fixé à une plaque de base (10) et la plaque de base (10) est fixée à une structure d'ossature via des trous passants (12) dans la plaque de base (10), et dans lequel avant l'étape c) et après l'étape b), la plaque de base (10) est détachée de la structure d'ossature, après quoi la plaque de base est tournée horizontalement ou verticalement, et après quoi la plaque de base (10) est à nouveau fixée à la structure d'ossature en utilisant les trous passants (12).
